# EUROPEAN PATENT SPECIFICATION

(11) **EP 2 542 224 B1**
(45) Date of publication and mention of the grant of the patent: **13.08.2014**
(21) Application number: 11704996.5
(22) Date of filing: 28.02.2011
(51) Int. Cl.: A61K 31/4184, A61K 31/4402, A61K 9/20, A61K 9/48

(54) **DABIGATRAN ETEXILATE-CONTAINING ORAL PHARMACEUTICAL COMPOSITION**
DABIGATRANEXILAT ENTHALTENDE ORALE PHARMAZEUTISCHE ZUSAMMENSETZUNG
COMPOSITION PHARMACEUTIQUE À VOIE ORALE CONTENANT D'ÉXILATE DE DABIGATRANE

(30) Priority: 01.03.2010 EP 10155059
(43) Date of publication of application: 09.01.2013
(73) Proprietor: Ratiopharm GmbH, 89079 Ulm (DE)
(72) Inventor: BRUECK, Sandra, 81479 München (DE); PAETZ, Jana, 53177 Bonn (DE); KOEBERLE, Martin, 81379 München (DE); STROHMEYER, Jutta, 81476 München (DE)
(74) Representative: Teipel, Stephan
(86) International application number: PCT/EP2011/052919
(87) International publication number: WO 2011/107427

(56) References cited:
- WO-A1-03/074056
- WO-A1-2010/007016
- WO-A1-2010/020602
- WO-A2-2008/009639

## Description

The present invention relates to an oral pharmaceutical composition containing dabigatran etexilate or a pharmaceutically acceptable salt thereof as active ingredient.

WO03074056, WO2008009639, WO2010020602 and WO2010007016 disclose dabigatran etexilate formulations.

Dabigatran etexilate (3-[(2-{[4-(hexyloxycarbonylamino-imino-methyl)-phenylamino]-methyl}-1-methyl-1*H*-benzimidazole-5-carbonyl)-pyridine-2-yl-amino]-propionic acid ethyl ester) has the following chemical formula:

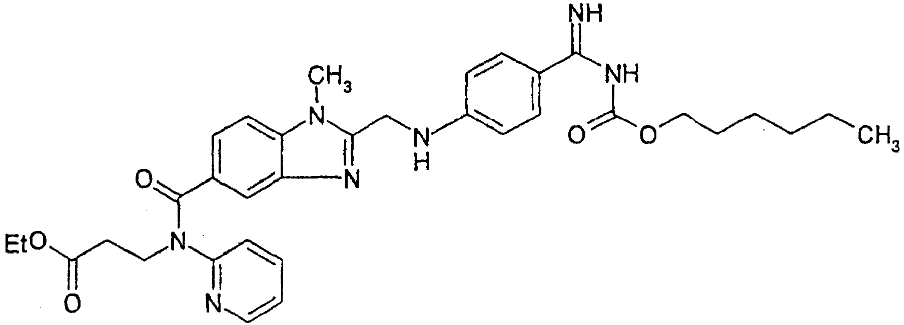

This active ingredient is already known from WO 98/37075. The main indication field of said active ingredient is the postoperative prophylaxis of deep venous thromboses and the prophylaxis of strokes.

The solubility of the active ingredient in water is only 1.8 mg/ml. Moreover, the active ingredient has a strong pH-dependent solubility that is greatly increased in the acidic environment. This leads to the problem that conventional oral pharmaceutical compositions have large variations in the bioavailability since the solubility of the active ingredient depends on the pH value in the patient's stomach. This is particularly problematic with patients in whom the stomach pH value is changed by physiological variability, illness, or premedications (for example, PP inhibitors). There is therefore a need for oral pharmaceutical compositions of the active ingredient dabigatran etexilate that provide a release that is independent from the pH value of the stomach and thus, provide bioavailability of the active ingredient.

WO 03/074056 suggests a pharmaceutical composition for oral application that comprises in addition to the active ingredient one or more pharmaceutically acceptable organic acids having a water solubility of >1 g/250 ml at 20°C. However, the corresponding pharmaceutical compositions may cause incompatibilities in the patient. Moreover, the addition of the organic acid restricts the possible amount of active ingredient in an appropriate tablet or capsule. This problem is further exacerbated by the fact that, as a rule, organic acids have only a low buffer capacity so that relatively large amounts of acid have to be added to cause a possible effect on the pH value of the ambience in dissolution of an appropriate tablet.

It has now surprisingly been found that these and further problems can be solved by the addition of an inorganic acidic excipient to a dabigatran etexilate-containing oral pharmaceutical composition. Thus, the present invention relates to an oral pharmaceutical composition comprising dabigatran etexilate or a pharmaceutically acceptable salt thereof, and an inorganic acidic excipient.

Without being bound by theory it is thought that the oral pharmaceutical composition therefore is better tolerated than the compositions known in the prior art since the inorganic acidic excipient is based on acids or salts that are already present in the body. In addition, inorganic acidic excipients often exhibit an only low molar weight so that the size of the dosage form can be reduced and the active ingredient load can be increased, respectively in comparison to conventional pharmaceutical compositions. This effect is enhanced by the fact that inorganic acidic excipients due to their high buffer capacity are able to absorb high intra-individual variations of the stomach pH value also in low amounts and thus, to ensure an uniform dissolution and influx of the active ingredient.

The inorganic acidic excipient employed in the oral pharmaceutical composition according to the invention should have a pH value in a 1% aqueous solution of <6, preferably a pH value in the range of from 1-4.

A suitable inorganic acidic excipient can be any pharmaceutically acceptable excipient wherein it may be especially an inorganic acid or an inorganic acidic salt. The amount of the employed inorganic acidic excipient can be chosen by the skilled person such that in dissolution of the oral pharmaceutical composition an acidic pH value is adjusted in the environment of the active ingredient. For example, the weight ratio of active ingredient to inorganic acidic excipient may be in the range of from 1:10 to 10:1.

Particularly suitable inorganic acidic excipients are inorganic acids such as hydrochloric acid, sulfuric acid, and phosphoric acid. Especially, in highly volatile acids such as hydrochloric acid it has proven to be advantageous if they are present also micro-encapsulated, adsorbed on a binder, or absorbed in a binder. Binders suitable for this are in particular polymers and silicic acid, especially pyrogenic silicic acid such as aerosil. As the polymers there can be advantageously employed hydrophilic polymers and in particular water-soluble polymers having a water solubility of >0.01 mg/ml. Microcrystalline cellulose is also suitable.

In general, the designation "hydrophilic polymer" comprises polymers with polar groups. Examples of polar groups are hydroxy, amino, carboxy, carbonyl, ethers, esters, and sulfonates. Hydroxy groups are particularly preferred.

Typically, the hydrophilic polymer has an average molecular weight in the range between 1000 and 250,000 g/mol, preferably 2000 and 100,000 g/mol, and particularly preferred between 4000 and 85,000 g/mol. Further, a 2% (w/w) solution of the hydrophilic polymer in pure water has preferably a viscosity between 2 and 8 mPas at 25°C. The viscosity is determined in accordance to the European Pharmacopoeia (Ph. Eur.), 6th edition, section 2.2.10.

Further, the hydrophilic polymer has preferably a glass transition temperature (Tg) between 20°C and 220°C, preferably 25°C to 160°C. The glass transition temperature (Tg) is the temperature at which the hydrophilic polymer becomes brittle on cooling and soft on heating. That means that the hydrophilic polymer becomes soft above the glass transition temperature and can be plastically deformed without breaking. The glass transition temperature is determined by means of a Mettler-Toledo® DSC 1 using a heating rate of 10°C/min. and a cooling rate of 15°C/min.

Examples of suitable hydrophilic polymers are cellulose derivatives, in particular hydrophilic derivatives of the cellulose (e.g. HPMC, HPC, carboxymethylcellulose, preferably as sodium or calcium salt, hydroxyethylcellulose, hydroxypropylcellulose), polyvinylpyrrolidone, preferably with a molecular weight of from 10,000 to 60,000 g/mol, copolymers of PVP, preferably co-polymers comprising vinylpyrrolidone and vinylacetate units (e.g. povidone, VA64, BASF), preferably with a molecular weight between 40,000 and 70,000 g/mol, poly(oxyethylene) alkyl ether, polyethylene glycol, co-block polymers of ethylene oxide, and propylene oxide (poloxamer, pluronic), derivatives of polymethacrylates, polyvinyl alcohol, polyvinyl alcohol derivatives, polyethylene glycol, and polyethylene glycol derivatives.

For the preparation of appropriate adsorbates or absorbates from the inorganic acid and the binder the acid can for example be sprayed onto the binder or rather granulated, or the binder can be dispersed in a solution of the acid. Alternatively, a solution/suspension of acid and binder can be commonly spray dried or lyophilized, for example.

As an alternative to the inorganic acid an inorganic acidic salt may be used as the inorganic acidic excipient. As inorganic acidic salt any pharmaceutically acceptable salt such as, for example hydrogen and dihydrogenphosphates, hydrogensulfates, ammonium chloride, ammonium sulfate, magnesium sulfate, magnesium chloride, ferrous chloride, ferric chloride, calcium chloride, and calcium sulfate is suitable. Hydrogen and dihydrogenphosphates and hydrogensulfates are in particular alkali or ammonium salts, especially sodium, potassium, and ammonium salts. The salts mentioned include their solvates, especially hydrates, such as for example magnesium chloride hexahydrate, calcium chloride mono or dihydrate, calcium sulfate dihydrate, magnesium sulfate monohydrate, and ferric chloride hexahydrate.

The inorganic salt should be water-soluble, wherein water-soluble salts are those having a solubility of >0.01 mg/ml. Further, mixtures of one or more inorganic acids and/or one or more inorganic acidic salts can be employed in the oral pharmaceutical composition according to the invention.

The inorganic acidic salt may either directly be mixed with the active ingredient and processed into appropriate pharmaceutical compositions or the salt can be prepared during the preparation of the pharmaceutical composition by adding an acid and a base. For example, suitable amounts of phosphoric acid and sodium or potassium hydroxide may be added to obtain a potassium phosphate buffer as the inorganic acidic salt.

Alternatively, also the inorganic acidic salt may be present adsorbed on a binder or absorbed in a binder. Suitable binders are those mentioned above for the inorganic acids, wherein appropriate adsorbates and absorbates also may be obtained in accordance to the methods mentioned above for the inorganic acids.

A particularly suitable pharmaceutically acceptable salt of the dabigatran etexilate is the mesylate salt, i.e. the salt of the methanesulfonic acid.

The high buffer capacity and the low molar mass of the inorganic acidic excipients employed according to the invention permit the preparation of oral pharmaceutical compositions with a high active ingredient load. Thus, in a particularly preferred embodiment the oral pharmaceutical composition according to the invention contains more than 45% by weight, preferably more than 50% by weight dabigatran etexilate or a pharmaceutically acceptable salt thereof based on the total weight of the composition.

Due to the acidic nature of some of the employed inorganic acidic excipients it may be advantageous to spatially separate these excipients in the pharmaceutical composition from the active ingredient. For example, this can be achieved by micro-encapsulation of the inorganic acid. In an alternative embodiment it is possible that the inorganic acidic excipient is present in a core material consisting of or containing the excipient and that the core material is surrounded by an active ingredient-containing layer. Additionally, the core material and the active ingredient-containing layer can be separated from each other by an interlayer. Correspondingly build up pharmaceutical compositions are described in WO 03/074056 in more detail.

For example, the oral pharmaceutical composition according to the invention may be present in the form of a capsule or a tablet.

In addition to the optionally present hydrophilic polymer the pharmaceutical composition can contain one or more further pharmaceutically acceptable excipients such as e.g. fillers, lubricants, flow control agents, release agents, and disintegrants. ("Lexikon der Hilfsstoffe fur Pharmazie, Kosmetik und angrenzende Gebiete", edited by H. P. Fiedler, 4th edition and "Handbook of Pharmaceutical Excipients", 3rd edition, edited by Arthur H. Kibbe, American Pharmaceutical Association, Washington, USA, and Pharmaceutical Press, London).

Fillers: The pharmaceutical composition can contain one or more filler(s). In general, a filler is a substance that increases the bulk volume of the mixture and thus the size of the resulting dosage form. Preferred examples of fillers are lactose and calcium hydrogenphosphate. The filler may be present in an amount of 0 to 80% by weight, preferred between 10 and 60% by weight of the total weight of the composition.

Lubricants: The function of the lubricant is to ensure that the pelletizing and the ejection take place without much friction between the solids and the walls. Preferably, the lubricant is an alkaline-earth metal stearate or a fatty acid, such as stearic acid. Typically, the lubricant is present in an amount of 0 to 2% by weight, preferably between 0.5 and 1.5% by weight of the total weight of the pharmaceutical composition.

Disintegrants: Usually, by a disintegrant is meant a substance that is capable of breaking up the tablet into smaller pieces as soon as it is in contact with a liquid. Preferred disintegrants are croscarmellose sodium, sodium carboxymethyl starch, cross-linked polyvinylpyrrolidone (crospovidon) or sodium carboxymethyl glycolate (e.g. explotab) and sodium bicarbonate. Typically, the disintegrants is present in an amount of 0 to 20% by weight, preferably between 1 and 15% by weight of the total weight of the composition.

Flow control agents: As the flow control agent there can be used e.g. colloidal silica. Preferably the flow control agent is present in an amount of 0 to 8% by weight, more preferably in an amount between 0.1 and 3% by weight of the total weight of the composition.

Release agents: The release agent can be e.g. talcum and is present in an amount between 0 and 5% by weight, preferably in an amount between 0.5 and 3% by the weight of the composition.

Moreover, the present invention relates to a method for the preparation of an oral pharmaceutical composition as described above which comprises mixing the active ingredient with the inorganic acidic excipient and optionally after further processing steps compressing the mixture to tablets or filling the mixture into capsules. In this method, the inorganic acidic excipient is preferably micro-encapsulated, adsorbed onto a binder, or absorbed into a binder before mixing. For that, the inorganic acidic excipient may be for example mixed in solution with the binder or the binder may be dissolved in the buffer solution and dried subsequently. Drying can be carried out by spray drying, lyophilization, or granulation onto a carrier.

Now, the present invention is explained in more detail with respect to the following examples without these should be interpreted as being limiting.

### Example 1:

| | |
|---|---|
| Dabigatran etexilate mesylate | 86.55 mg |
| Avicel 102 | 78 mg |
| Sodium dihydrogenphosphate | 55.00 mg |
| Phosphoric acid | q.s. |
| HPMC | 18 mg |
| Kollidon CL | 8 mg |
| Aerosil | 1 mg |
| Magnesium stearate | 1.5 mg |

### Example 2:

| | |
|---|---|
| Dabigatran etexilate mesylate | 86.55 mg |
| Dicafos | 20 mg |
| Ammonium dihydrogenphosphate | 70.00 mg |
| Phosphoric acid | q.s. |
| Povidon 25 | 5 mg |
| Kollidon CL | 8 mg |
| Aerosil | 1 mg |
| Magnesium stearate | 1.5 mg |

In the examples 1 and 2 the inorganic acidic salts are dissolved in water and subsequently the pH value of the solution is adjusted to less than 3 with phosphoric acid. In this solution the polymer is dissolved and subsequently spray dried/lyophilized. The prepared intermediate is mixed with the active ingredient, filler, blasting agent, and flow improver for 15 min. on the tumbler. After adding the lubricant it is again mixed for 5 min. The final mixture can be compressed to tablets or filled into capsules.

Alternatively, the active ingredient can already be added to the manufacturing process of the intermediates or the preparation may be transferred to a granule method. Here, the pH controlled polymer solution was used to granulate filler and active ingredient. The dried granulate was sieved over 0.71 mm and subsequently mixed with disintegrant, flow improver for 15 min. on a tumbler. After adding the lubricant it was mixed for another 5 min. The final mixture can be compressed to tablets or alternatively filled into capsules.

### Example 3:

| | |
|---|---|
| Dabigatran etexilate mesylate | 86.55 mg |
| Lactose monohydrate | 60 mg |
| Potassium dihydrogenphosphate | 65.00 mg |
| Phosphoric acid | q.s. |
| Croscarmellose | 8 mg |
| Aerosil | 1 mg |
| Magnesium stearate | 1.5 mg |

The inorganic acidic salt is dissolved in water and subsequently the pH value of the solution is adjusted to less than 3 with phosphoric acid. Subsequently, the solution is spray dried/lyophilized. The prepared intermediate is mixed with the filler, disintegrant, and flow improver for 15 min. on the tumbler. After adding the lubricant it is again mixed for 5 min. The final mixture can be compressed to tablets or filled into capsules.

Alternatively, the active ingredient can already be added to the manufacturing process of the intermediate.

### Example 4:

| | |
|---|---|
| Dabigatran etexilate mesylate | 86.55 mg |
| Lactose anhydrate | 55 mg |
| Magnesium chloride hexahydrate | 55.00 mg |
| Croscarmellose | 8 mg |
| Aerosil | 1 mg |
| Magnesium stearate | 1.5 mg |

All substances except the magnesium stearate are mixed for 15 min. in the tumbler. After adding the magnesium stearate it is mixed for another 5 min. The final mixture can be compressed to tablets or filled into capsules.

### Example 5:

| | |
|---|---|
| Dabigatran etexilate mesylate | 86.55 mg |
| Microcrystalline cellulose (MCC) | 80 mg |
| Povidon 25 | 15.00 mg |
| Hydrochloric acid | q.s. |
| Kollidon CL | 8 mg |
| Aerosil | 1 mg |
| Magnesium stearate | 1.5 mg |

An aqueous solution of Povidon 25 is adjusted to pH 1 with hydrochloric acid. With this granulation solution MCC is granulated in the fluid bed granulator. The dried granulate is mixed with Kollidon CL and Aerosil for 10 min. in the tumbler. After adding the magnesium stearate it is mixed for another 5 min. The final mixture can be compressed to tablets or filled into capsules.

## Claims

1. An oral pharmaceutical composition comprising dabigatran etexilate or a pharmaceutical acceptable salt thereof and an inorganic acidic excipient.

2. The oral pharmaceutical composition according to claim 1, wherein the inorganic acidic excipient has a pH value in a 1% aqueous solution of <6.

3. The oral pharmaceutical composition according to any one of the preceding claims, wherein the inorganic acidic excipient is an inorganic acid and/or an inorganic acidic salt.

4. The oral pharmaceutical composition according to claim 3, wherein the inorganic acid is selected from hydrochloric acid, sulfuric acid, and phosphoric acid.

5. The oral pharmaceutical composition according to claim 4, wherein the inorganic acid is present micro-encapsulated, adsorbed on a binder or absorbed in a binder.

6. The oral pharmaceutical composition according to claim 5, wherein the binder is selected from polymers, in particular hydrophilic polymers, and silicic acid.

7. The oral pharmaceutical composition according to claim 3, wherein the inorganic acidic salt is selected from hydrogen and dihydrogenphosphates, hydrogensulfates, ammonium chloride, ammonium sulfate, magnesium sulfate, magnesium chloride, ferrous chloride, ferric chloride, calcium chloride, and calcium sulfate.

8. The oral pharmaceutical composition according to claim 7, wherein the hydrogen and dihydrogenphosphates and hydrogensulfates are alkali or ammonium salts.

9. The oral pharmaceutical composition according to claim 7 or 8, wherein the inorganic acidic salt is present adsorbed on a binder or absorbed in a binder.

10. The oral pharmaceutical composition according to claim 9, wherein the binder is selected from polymers, in particular hydrophilic polymers, and silicic acid.

11. The oral pharmaceutical composition according to any one of the preceding claims, wherein the active ingredient is present as mesylate salt.

12. The oral pharmaceutical composition according to any one of the preceding claims containing more than 45% by weight dabigatran etexilate or a pharmaceutically acceptable salt thereof based on the total weight of the composition.

13. The oral pharmaceutical composition according to any one of the preceding claims comprising a core material consisting of or containing the inorganic acidic excipient .and an active ingredient-containing layer surrounding the core material.

14. The oral pharmaceutical composition according to any one of the preceding claims, wherein it is a capsule or a tablet.

15. A method for the preparation of an oral pharmaceutical composition according to one of claims 1 to 14 which comprises mixing the active ingredient with the inorganic acidic excipient and optionally after further processing steps compressing the mixture to tablets or filling the mixture into capsules.

16. The method according to claim 15, wherein the inorganic acidic excipient is micro-encapsulated, adsorbed onto a binder or absorbed into a binder before mixing.

## Patentansprüche

1. Orale pharmazeutische Zusammensetzung, umfassend Dabigatranetexilat oder ein pharmazeutisch verträgliches Salz davon und einen anorganischen, sauren Hilfsstoff.

2. Orale pharmazeutische Zusammensetzung nach Anspruch 1, wobei der anorganische, saure Hilfsstoff in 1 %-iger wässriger Lösung einen pH-Wert von <6 aufweist.

3. Orale pharmazeutische Zusammensetzung nach einem der vorhergehenden Ansprüche, wobei der anorganische, saure Hilfsstoff eine anorganische Säure und/oder ein anorganisches, saures Salz ist.

4. Orale pharmazeutische Zusammensetzung nach Anspruch 3, wobei die anorganische Säure ausgewählt ist aus Salzsäure, Schwefelsäure und Phosphorsäure.

5. Orale pharmazeutische Zusammensetzung nach Anspruch 4, wobei die anorganische Säure mikroverkapselt, auf einem Bindemittel adsorbiert oder in einem Bindemittel absorbiert vorliegt.

6. Orale pharmazeutische Zusammensetzung nach Anspruch 5, wobei das Bindemittel ausgewählt ist aus Polymeren, insbesondere hydrophilen Polymeren, und Kieselsäure.

7. Orale pharmazeutische Zusammensetzung nach Anspruch 3, wobei das anorganische, saure Salz ausgewählt ist aus Hydrogen- und Dihydrogenphosphaten, Hydrogensulfaten, Ammoniumchlorid, Ammoniumsulfat, Magnesiumsulfat, Magnesiumchlorid, Eisen(II)-chlorid, Eisen(III)-chlorid, Calciumchlorid und Calciumsulfat.

8. Orale pharmazeutische Zusammensetzung nach Anspruch 7, wobei es sich bei den Hydrogen- und Dihydrogenphosphaten und Hydrogensulfaten um Alkali- oder Ammoniumsalze handelt.

9. Orale pharmazeutische Zusammensetzung nach Anspruch 7 oder 8, wobei das anorganische, saure Salz auf einem Bindemittel adsorbiert oder in einem Bindemittel absorbiert vorliegt.

10. Orale pharmazeutische Zusammensetzung nach Anspruch 9, wobei das Bindemittel ausgewählt ist aus Polymeren, insbesondere hydrophilen Polymeren, und Kieselsäure.

11. Orale pharmazeutische Zusammensetzung nach einem der vorhergehenden Ansprüche, wobei der Wirkstoff als Mesylatsalz vorliegt.

12. Orale pharmazeutische Zusammensetzung nach einem der vorhergehenden Ansprüche, die mehr als 45 Gew.-% Dabigatranetexilat oder ein pharmazeutisch verträgliches Salz davon enthält, bezogen auf das Gesamtgewicht der Zusammensetzung.

13. Orale pharmazeutische Zusammensetzung nach einem der vorhergehenden Ansprüche, umfassend ein Kernmaterial, das aus dem anorganischen sauren Hilfsstoff besteht oder diesen enthält, und eine wirkstoffhaltige Schicht, die das Kernmaterial umschließt.

14. Orale pharmazeutische Zusammensetzung nach einem der vorhergehenden Ansprüche, wobei es sich um eine Kapsel oder eine Tablette handelt.

15. Verfahren zur Herstellung einer oralen pharmazeutischen Zusammensetzung gemäß einem der Ansprüche 1 bis 14, umfassend das Mischen des Wirkstoffs mit dem anorganischen, sauren Hilfsstoff und, gegebenenfalls nach weiteren Verarbeitungsschritten, das Verpressen der Mischung zu Tabletten oder das Füllen der Mischung in Kapseln.

16. Verfahren nach Anspruch 15, wobei der anorganische, saure Hilfsstoff vor dem Mischen mikroverkapselt, auf ein Bindemittel adsorbiert oder in ein Bindemittel absorbiert wird.

## Revendications

1. Composition pharmaceutique à voie orale comprenant de l'étéxilate de dabigatran ou un sel pharmaceutiquement acceptable de celui-ci et un excipient acide inorganique.

2. Composition pharmaceutique à voie orale selon la revendication 1, dans laquelle l'excipient acide inorganique présente une valeur de pH < 6 dans une solution aqueuse à 1%.

3. Composition pharmaceutique à voie orale selon l'une quelconque des revendications précédentes, dans laquelle l'excipient acide inorganique est un acide inorganique et/ou un sel d'acide inorganique.

4. Composition pharmaceutique à voie orale selon la revendication 3, dans laquelle l'acide inorganique est choisi parmi l'acide chlorhydrique, l'acide sulfurique, et l'acide phosphorique.

5. Composition pharmaceutique à voie orale selon la revendication 4, dans laquelle l'acide inorganique est présent sous forme microencapsulée, adsorbée sur un liant ou absorbée dans un liant.

6. Composition pharmaceutique à voie orale selon la revendication 5, dans laquelle le liant est choisi parmi les polymères, en particulier les polymères hydrophiles, et l'acide silicique.

7. Composition pharmaceutique à voie orale selon la revendication 3, dans laquelle le sel d'acide inorganique est choisi parmi les hydrogéno et les dihydrogénophosphates, les hydrogénosulfates, le chlorure d'ammonium, le sulfate d'ammonium, le sulfate de magnésium, le chlorure de magnésium, le chlorure ferreux, le chlorure ferrique, le chlorure de calcium, et le sulfate de calcium.

8. Composition pharmaceutique à voie orale selon la revendication 7, dans laquelle les hydrogéno et les dihydrogénophosphates et les hydrogénosulfates sont des sels alcalins ou des sels d'ammonium.

9. Composition pharmaceutique à voie orale selon la revendication 7 ou 8, dans laquelle le sel d'acide inorganique est présent sous forme adsorbée sur un liant ou absorbée dans un liant.

10. Composition pharmaceutique à voie orale selon la revendication 9, dans laquelle le liant est choisi parmi les polymères, en particulier les polymères hydrophiles, et l'acide silicique.

11. Composition pharmaceutique à voie orale selon l'une quelconque des revendications précédentes dans laquelle l'ingrédient actif est présent en tant que sel de mésylate.

12. Composition pharmaceutique à voie orale selon l'une quelconque des revendications précédentes contenant plus de 45% en poids d'étéxilate de dabigatran ou un sel pharmaceutiquement acceptable de celui-ci sur base du poids total de la composition.

13. Composition pharmaceutique à voie orale selon l'une quelconque des revendications précédentes comprenant un matériau noyau consistant en ou contenant l'excipient acide inorganique et une couche contenant l'ingrédient actif entourant le matériaux central.

14. Composition pharmaceutique à voie orale selon l'une quelconque des revendications précédentes, dans laquelle il s'agit d'une capsule ou d'un comprimé.

15. Procédé de préparation d'une composition à voie orale selon l'une quelconque des revendications 1 à 14 qui comprend un mélange de l'ingrédient actif avec l'excipient acide inorganique et optionnellement suite à des étapes de fabrication en plus une compression du mélange pour obtenir des comprimés ou un remplissage du mélange dans les capsules.

16. Procédé selon la revendication 15, dans lequel l'excipient acide inorganique est microencapsulé, adsorbé sur un liant ou absorbé dans un liant avant mélange.
